# DEMANDE DE BREVET EUROPEEN

(11) **EP 0 839 545 A1**
(43) Date de publication de la demande: **06.05.1998**
(21) Numéro de dépôt: 97402494.5
(22) Date de dépôt: 21.10.1997
(51) Int. Cl.: A61M 16/00

(54) **Dispositif d'assistance respiratoire**

(30) Priorité: 30.10.1996 FR 9613265
(71) Demandeur: TAEMA, F-92160 Antony (FR)
(72) Inventeur: Ruton, Stéphane, 78220 Viroflay (FR)
(74) Mandataire: Le Moenner, Gabriel

(57) **Abrégé**

L'invention concerne un dispositif d'assistance respiratoire comprenant :
- une branche inspiratoire (2) reliée en permanence, à son extrémité amont, à une première source de débit (1) de gaz sous pression et, à son extrémité aval, aux voies respiratoires d'un utilisateur,
- au moins une première (9) et une deuxième (3) valves expiratoires agencées sur ladite branche respiratoire (2) commandées pour être fermées pendant la phase inspiratoire,
- un moyen de détection (10) détectant, à proximité de l'extrémité aval de la branche inspiratoire, l'activité respiratoire de l'utilisateur et transmettant une donnée d'activité respiratoire à des moyens de pilotage (12), lesdits moyens de pilotage (12) commandant la première source de débit (1) de gaz de manière à délivrer, dans la branche inspiratoire (2), un flux de gaz à débit non nul et sensiblement constant pendant toute la phase expiratoire.

## Description

La présente invention concerne un dispositif d'assistance respiratoire à deux niveaux de pression, utilisable dans le cadre d'une assistance respiratoire à domicile ou en milieu hospitalier.

Classiquement, l'assistance respiratoire consiste à ventiler un patient à l'aide d'un gaz sous pression, par exemple de l'air ou de l'air enrichi en oxygène, c'est-à-dire à appliquer pendant la phase inspiratoire, initiée habituellement par le patient, une pression positive constante dans le circuit "patient" d'un appareil respiratoire.

Le circuit "patient" est habituellement constitué d'éléments de canalisation permettant de relier les voies aériennes du patient avec la source de gaz sous pression; le circuit patient comprend donc des éléments, tels le ou les conduits respiratoires, un masque ou des lunettes respiratoires, une sonde de trachéotomie.

La pression régnant dans le circuit patient varie en fonction de la phase respiratoire: phase inspiratoire ou phase expiratoire. En effet, durant la phase inspiratoire, le gaz sous pression issu d'une source de débit de gaz, communément appelée turbine, est distribué aux voies respiratoires du patient à une pression inspiratoire donnée, alors que durant la phase expiratoire, l'expiration initiée par le patient est passive et s'effectue à la pression atmosphérique ou à une pression expiratoire positive donnée, encore appelée PEP; l'échappement des gaz expirés s'effectuant au travers soit d'une valve (ou plusieurs) expiratoire aménagée sur le circuit patient, soit au travers de trous ou d'ouïes aménagés dans le masque respiratoire.

Les dispositifs d'assistance respiratoire fonctionnant sur ce principe sont communément appelés dispositifs d'assistance respiratoire à deux niveaux de pression.

On a constaté qu'un échappement des gaz expiratoires riches en CO₂, via des trous ou des ouïes pratiqués dans le masque respiratoire, présentait l'inconvénient majeur de ne pas permettre une évacuation totale des gaz expirés, qui ont tendance à s'accumuler dans certaines parties du circuit patient, en particulier dans le masque respiratoire. Une telle accumulation conduit, durant la phase inspiratoire suivante, à une réinhalation des gaz riches en CO₂, ce qui est nuisible pour le patient.

Les dispositifs d'assistance respiratoire à deux niveaux de pression munis d'une ou plusieurs valves expiratoires sont donc préférés.

On peut citer EP-B-0317417, qui décrit un dispositif d'assistance à la respiration dans lequel le circuit patient comprend une valve d'expiration à commande pneumatique. Pendant la phase d'inspiration, l'entrée de commande de cette valve est soumise à la pression d'une source de débit pressurisée, ce qui ferme la valve d'expiration et permet de relier, de manière étanche, le circuit patient avec ladite source de débit de gaz pressurisé. Lorsqu'une diminution significative du débit inspiré est détecté, une électronique de commande interrompt totalement le fonctionnement de la source de débit pressurisé, dont la structure est telle que son orifice de sortie se trouve alors ramené à la pression atmosphérique, laquelle pression est appliquée à la valve d'expiration, lui permettant ainsi de s'ouvrir.

EP-A-0425092 a trait à un dispositif d'aide à la respiration comportant, à la place de la valve d'expiration, un orifice de fuite permanente, calibrée, tandis que l'on règle à deux niveaux différents la pression d'une source de débit, selon qu'on se trouve en phase d'inspiration ou d'expiration.

En d'autres termes, ce type de dispositifs connus de l'art antérieur a un fonctionnement basé sur une interruption au moins partielle, et généralement totale, de la source de débit de gaz durant la phase expiratoire. Or, tout arrêt de la source de débit de gaz présente un inconvénient majeur, à savoir que lors du passage d'une phase expiratoire à la phase inspiratoire suivante, le temps de montée en pression du circuit patient dépend étroitement, d'une part, de la puissance de la source de débit de gaz et, d'autre part, de l'inertie mécanique de celle-ci.

Il en résulte que pour être performants et efficaces, et pallier ces problèmes, ces dispositifs doivent nécessairement être dotés d'une source de débit de gaz puissante et à faible inertie mécanique. Or, les sources de débit de gaz, actuellement disponibles dans le commerce, qui permettent de répondre à de telles spécifications de puissance et d'inertie, présentent habituellement des inconvénients incompatibles avec une utilisation à domicile et, plus généralement, dans le domaine médical: encombrement excessif, nuisances sonores...

Par ailleurs, les dispositifs d'assistance respiratoire connus présentent aussi parfois un autre inconvénient, à savoir qu'ils ne permettent pas une évacuation parfaite du gaz expiré.

En effet, afin d'éviter que du gaz expiratoire riche en CO₂ ne remonte dans le circuit patient, pendant la phase expiratoire, et ne soit ensuite réinspiré lors de la phase inspiratoire suivante, il est recommandé de maintenir un écoulement de gaz, dans le circuit patient, entre la source de débit et le patient; cet écoulement permettant d'évacuer les gaz expirés.

On comprend aisément que si on interrompt totalement la communication entre la source de débit de gaz et le patient, durant la phase expiratoire, aucune circulation de gaz ne peut exister au sein du circuit patient et les gaz expiratoires risquent de s'accumuler.

Une solution partielle à ces problèmes est apportée par WO-A-94/06499, qui décrit un dispositif d'aide à la respiration comprenant un circuit patient ayant une branche inspiratoire reliée à une source de débit inspiratoire pressurisée et une branche expiratoire, dans laquelle est installée une valve d'expiration commandée pour être fermée pendant l'inspiration.

Selon, un premier mode de réalisation de ce dispositif, des moyens de pilotage commandent, d'une part, des moyens de distribution et, d'autre part, l'interruption de la communication entre la source de débit inspiratoire et la branche inspiratoire du circuit patient, lorsqu'un capteur détecte que le patient prépare une phase expiratoire; la communication entre la source de débit inspiratoire et la branche inspiratoire étant rétablie lors du passage en phase inspiratoire, par commande des moyens de distribution. En d'autres termes, pendant toute la phase expiratoire, la source de débit inspiratoire est maintenue en fonctionnement dans le sens tendant à fournir du débit inspiratoire.

On comprend aisément que ce mode de réalisation n'est pas satisfaisant car, pendant la phase expiratoire, la source de débit continue à distribuer du gaz sous pression, qui va s'accumuler dans le conduit patient entre ladite source de débit et le site d'interruption de la communication. De cette accumulation de gaz, il va résulter une surpression en amont du circuit patient, laquelle surpression va se propager, lors du passage en phase inspiratoire suivante et après rétablissement de ladite communication, jusqu'aux poumons du patient, qui subira alors un "choc respiratoire pulmonaire" néfaste, dû à cette surpression. Par ailleurs, le gaz distribué par la source de débit, durant la phase expiratoire, n'étant pas évacué, ladite source de débit est soumise à détérioration, notamment par échauffement. Enfin, ce mode de réalisation ne résout pas le problème d'accumulation des gaz expirés étant donné que la communication entre la source de débit et le masque respiratoire est interrompue durant la phase expiratoire.

Afin de tenter de pallier ces problèmes, WO-A-94/06499 propose un second mode de réalisation, mettant en oeuvre un conduit de compensation de fuite, permettant d'acheminer, durant la phase expiratoire, une partie du gaz délivré par la source respiratoire jusqu'en un site du circuit patient en aval du site d'interruption de communication. Toutefois, si cette solution permet de résoudre partiellement les problèmes de détérioration de la source de débit et d'évacuation des gaz expirés, elle présente, par contre, plusieurs autres inconvénients.

En effet, le conduit de dérivation a un diamètre constant non modulable et n'est donc pas adapté à toutes les valeurs de débit, par exemple en fonction du patient à ventiler: adulte ou enfant.

Ainsi, lorsque le débit de gaz délivré par la source de débit sera trop important pour que le conduit de dérivation ne puisse parfaitement remplir son rôle, du fait d'un diamètre trop faible par rapport audit débit, les problèmes précédents de surpression, de risques de "choc respiratoire pulmonaire" et de détérioration de la source de débit se poseront à nouveau. A l'inverse, lorsque le débit de gaz délivré sera inférieur à une certaine valeur seuil, le conduit de dérivation permettra le passage vers le patient, pendant la phase expiratoire, d'une trop grande quantité de gaz qui s'opposera à une expiration correcte et à une évacuation satisfaisante du gaz enrichi en CO₂ expiré par le patient.

En définitive, les solutions préconisées par ce document ne sont pas satisfaisantes. Il est donc nécessaire de développer de nouveaux matériels permettant de pallier les problèmes précédemment cités, lesquels ne présenteraient pas les inconvénients des dispositifs de l'art antérieur. La présente invention, s'inscrivant dans ce contexte, a donc pour but de proposer un dispositif d'assistance à la respiration à deux niveaux de compression, lequel permet de remédier aux inconvénients des dispositifs de l'art antérieur et, en particulier,:
- de minimiser au maximum le temps de montée en pression du circuit patient lors du passage d'une phase expiratoire à la phase inspiratoire suivante;
- de s'affranchir de toute interruption de communication dans le circuit patient durant les phases expiratoires;
- son utilisation tant dans l'assistance respiratoire à domicile qu'en milieu hospitalier;
- une évacuation efficace des gaz expirés lors de la phase expiratoire;
- d'éviter tout "choc respiratoire pulmonaire" lors du passage d'une phase expiratoire à une phase inspiratoire suivante;
- de s'affranchir de toute mise en place de conduits supplémentaires de compensation de fuites sur le circuit patient tout en étant de conception simple et de coût raisonnable.
La présente invention concerne alors un dispositif d'assistance respiratoire comprenant :
- une branche inspiratoire reliée en permanence, à son extrémité amont, à une première source de débit de gaz sous pression et, à son extrémité aval, aux voies respiratoires d'un utilisateur,
- au moins une première et une deuxième valves expiratoires agencées sur ladite branche respiratoire commandées pour être fermées pendant la phase inspiratoire,
- un moyen de détection détectant, à proximité de l'extrémité aval de la branche inspiratoire, l'activité respiratoire de l'utilisateur et transmettant une donnée d'activité respiratoire à des moyens de pilotage, lesdits moyens de pilotage commandant la première source de débit de gaz de manière à délivrer, dans la branche inspiratoire, un flux de gaz à débit non nul et sensiblement constant pendant toute la phase expiratoire.

De préférence, les moyens de pilotage commandent la première source de débit de gaz de manière à délivrer, dans la branche inspiratoire, un flux de gaz dont le débit, pendant une phase expiratoire, est sensiblement égal au débit délivré en fin de phase inspiratoire précédente.

Avantageusement, au moins une première valve expiratoire est agencée à proximité de l'extrémité amont de la branche inspiratoire et/ou au moins une deuxième valve expiratoire est agencée à proximité de l'extrémité aval de la branche inspiratoire.

Selon un mode de réalisation préféré, le dispositif de l'invention comporte, en outre, une deuxième source de débit de gaz sous pression et un moyen de distribution, ladite deuxième source de débit et ledit moyen de distribution étant commandés par les moyens de pilotage.

De manière préférée, le moyen de détection est un capteur de pression pouvant être installé dans le masque ou simplement connecté à celui-ci par le biais de moyens de prise de pression, les valves expiratoires sont des valves pneumatiques à ballonnets et/ou le moyen de distribution est une électrovanne.

Avantageusement, durant la phase inspiratoire, l'électrovanne établit la communication entre la première source de débit de gaz sous pression et les ballonnets des valves pneumatiques, de manière à fermer lesdites valves pendant toute la phase inspiratoire, et/ou durant la phase expiratoire, l'électrovanne établit la communication entre la deuxième source de débit de gaz sous pression et les ballonnets des valves pneumatiques, de manière à contrôler l'ouverture desdites valves.

Préférentiellement, le débit de la deuxième source de gaz est inférieur au débit de la première source de gaz sous pression.

Si besoin est, une partie du gaz délivré par la deuxième source de gaz sous pression est échappé à l'atmosphère via un échappement.

Avantageusement, le gaz sous pression est de l'air ou de l'air enrichi en oxygène.

Par ailleurs, l'invention concerne également un procédé de mise en oeuvre du dispositif selon l'une des revendications précédentes, caractérisé en ce que la tension de commande de la première source de débit est maintenue à une valeur sensiblement constante et non nulle pendant toute la phase expiratoire.

De préférence, la tension de commande de la première source de débit est maintenue, pendant toute la phase expiratoire, à une valeur sensiblement égale à la valeur qu'elle avait en fin de phase inspiratoire précédente.

Avantageusement, l'expiration est réalisée sous pression expiratoire positive.

L'invention va maintenant être décrite plus en détail à l'aide d'un mode de réalisation de l'invention, en référence aux figures annexées, données à titre illustratif, mais non limitatif.

La figure 1 représente le schéma d'un mode de réalisation d'un dispositif d'assistance respiratoire conforme à l'invention.

Les figures 2 et 3 représentent des organigrammes de fonctionnement d'un dispositif d'assistance respiratoire conforme à l'invention, lors d'une phase inspiratoire et lors d'une phase expiratoire, respectivement.

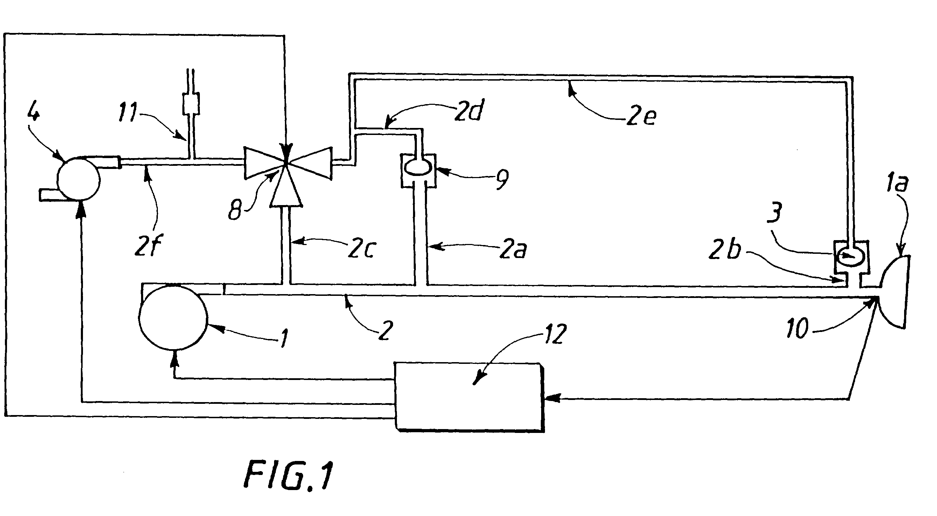

Un dispositif d'assistance respiratoire selon l'invention est représenté sur la figure 1.

Sur cette figure 1, la branche respiratoire 2 est reliée par l'une de ses extrémités à une première source de débit 1 de gaz sous pression, telle une turbine délivrant un gaz à une pression par exemple dans la gamme 0 à 50 mbar, et par son autre extrémité aux voies respiratoires du patient par l'intermédiaire d'un masque respiratoire la. Deux valves 3 et 9 à ballonnets sont agencées sur la branche inspiratoire 2, de manière à être fermées durant les phases inspiratoires pour éviter que le gaz sous pression issu de la première source de débit de gaz 1 ne soit envoyé vers l'atmosphère.

La valve à ballonnet 9 est agencée en amont de la branche inspiratoire 2, c'est-à-dire à proximité de la source 1 de débit de gaz sous pression, alors que la valve à ballonnet 3 est agencée, quant à elle, en aval de la branche inspiratoire 2, c'est-à-dire le plus près possible des voies respiratoires du patient, par exemple au niveau du masque respiratoire la.

Le dispositif représenté sur la figure 1 comporte également une électrovanne 8 destinée à établir une communication entre la branche inspiratoire 2, via une dérivation 2c, et les conduits 2d et 2e reliés aux ballonnets des valves 9 et 3, respectivement, de manière à permettre leur fermeture durant les phases inspiratoires.

Durant les phases expiratoires, l'électrovanne 8 est pilotée par les moyens de pilotage 12, de façon à établir la communication entre les ballonnets 9 et 3, via les conduites 2d et 2e respectivement, et une deuxième source de débit 4 de gaz délivrant du gaz à une pression par exemple dans la gamme 0 à 10 mbar, via le conduit 2f. Ainsi, étant donné que la deuxième source de débit 4 est également commandée par les moyens de pilotage 12, il est possible de contrôler facilement la pression dans les ballonnets des valves 9 et 3, et par la même, la pression d'expiration positive (PEP), c'est-à-dire la pression minimale devant être exercée par le patient (lors de l'expiration) pour permettre une mise à l'atmosphère de la branche inspiratoire 2, par ouverture des valves 9 et 3 pendant les phases expiratoires. Un conduit d'échappement à l'atmosphère 11 est aménagé sur le conduit 2f entre l'électrovanne 8 et la deuxième source de débit 4, communément appelée "pompe", afin d'évacuer vers l'atmosphère:
- durant les phases inspiratoires, le gaz distribué de manière continue par la deuxième source de débit 4;
- durant les phases expiratoires, tout excès de gaz dans les conduites 2f, 2d ou 2e, afin d'éviter une surpression éventuelle dans les ballonnets 9 et 3 et donc une PEP trop élevée et nuisible au patient.

La première source de débit 1 de gaz sous pression, la deuxième source de débit 4 et l'électrovanne 8 sont pilotées par des moyens de pilotage 12, lesquels sont reliés à un capteur de pression 10 aménagé dans/ou à proximité du masque respiratoire la.

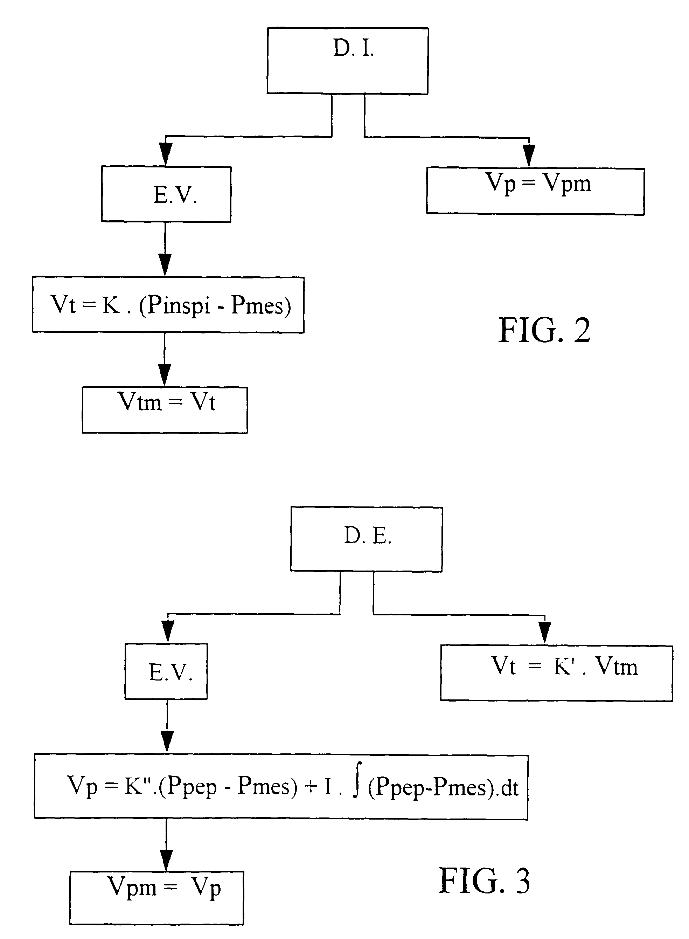

Le fonctionnement de moyens de pilotage 12 pendant les différentes phases respiratoires est décrit en détail ci-après, en référence aux figures 2 et 3.

Le type de phase respiratoire, à savoir, phase inspiratoire ou phase expiratoire, est déterminé à l'aide du capteur 10, lequel transmet un signal de phase respiratoire (respiratoire ou expiratoire) aux moyens de pilotage 12. A titre d'exemple, on peut utiliser un capteur du type SENSYM© (O à 20 mbar) ou tout autre dispositif de détection de phase respiratoire, tel celui décrit dans EP 0505232.

Pendant toute la durée de la phase inspiratoire, le capteur 10 mesure la pression d'inspiration, laquelle va être comparée à une valeur de pression d'inspiration préfixée. Tant que la différence entre la valeur de pression d'inspiration préfixée et la valeur de pression d'inspiration mesurée reste proportionnelle à une valeur donnée correspondant à une valeur de pression de phase inspiratoire, les moyens de pilotage 12 commandent la première source de débit 1, la deuxième source de débit 4 et l'électrovanne 8, de manière à:
- maintenir une communication entre la branche inspiratoire 2 et les ballonnets des vannes 9 et 3 via les conduites 2c, 2d et 2e, afin de maintenir lesdites valves 9 et 3 fermées;
- maintenir constante la tension de commande de la première source de débit 1 afin de délivrer aux voies respiratoires du patient du gaz sous pression, via la branche inspiratoire 2;
- et de maintenir la tension de commande de la deuxième source de débit 4 à une valeur constante, au choix, à une valeur nulle ou à une valeur préfixée non nulle. Dans le cadre de la présente invention, une valeur préfixée non nulle est préférée.

Ce mode de fonctionnement est schématisé sur l'organigramme de la figure 2. Plus précisément, lors de la détection de l'inspiration (D. I.), c'est-à-dire d'un signal inspiratoire, on assiste, d'une part, au maintien de la tension de commande (Vp) de la deuxième source de débit 4 à la valeur moyenne de tension (Vpm) de celle-ci mémorisée lors de la phase expiratoire précédente, et, d'autre part, après basculement de l'électrovanne(E. V.) 8, au maintien de la tension de commande (Vt) de la première source de débit 1 à une valeur proportionnelle (coefficient K) à la différence entre la valeur de consigne de pression (Pinspi) pendant la phase inspiratoire et la valeur de pression mesurée (Pmes); la valeur moyenne de la tension de commande (Vtm) de la première source de débit mémorisée étant alors égale à Vt.

Lorsque la différence entre la pression d'inspiration réglée et la pression d'inspiration mesurée correspond à une valeur préfixée de fin de phase inspiratoire, les moyens de pilotage 12 interrompent la communication entre la branche inspiratoire 2 et les ballonnets des valves 9 et 3, et établissent la communication entre lesdits ballonnets et la deuxième source de débit 4 afin de permettre une régulation de la quantité de gaz emprisonné dans lesdits ballonnets, et donc de la PEP. Cette régulation de pression est contrôlée par les moyens de pilotage 12, lesquels agissent sur la deuxième source de débit 4. Pour éliminer la surpression existant dans les ballonnets 9 et 3 durant la phase inspiratoire (valves fermées), les moyens de pilotage agissent sur la deuxième source de débit 4 de manière à diminuer son débit; l'excès de gaz (surpression) des ballonnets 9 et 3 pouvant alors s'échapper à l'atmosphère via l'échappement 11, ce qui permet une ouverture plus facile des vannes à ballonnets 9 et 3, due à une PEP plus basse.

En outre, les moyens de pilotage 12 mémorisent, en fin de phase inspiratoire, la valeur de la tension de commande de la première source de débit 1.

Ensuite, durant la phase expiratoire, la tension de commande de la première source de débit 1 est maintenue constante à ladite valeur qu'elle avait en fin de phase inspiratoire précédente.

L'ouverture des valves 9 et 3 à ballonnets est régulée de manière à permettre au gaz sous pression de s'échapper de la branche inspiratoire 2 par la valve 9, via la conduite 2a et, de façon moindre, par la valve à ballonnets 3 via la conduite 2b. Les gaz riches en CO₂ expirés par le patient sont, quant à eux, évacués, à PEP contrôlée, par la valve à ballonnets 3, via la conduite 2b ; ils ne peuvent donc pas stagner ou remonter dans la branche inspiratoire.

Les moyens de pilotage 12 commandent également la tension de la deuxième source de débit 4. De la même façon que précédemment, lors du passage de la phase inspiratoire à la phase expiratoire, lorsque le capteur 10 détecte une valeur de pression donnée correspondant au passage de la phase expiratoire à la phase inspiratoire suivante, il transmet cette information aux moyens de pilotage 12 qui vont alors:
- rétablir la communication entre la branche inspiratoire 2 et les ballonnets des valves 9 et 3 au moyen de l'électrovanne 8, ce qui a pour conséquence de faire augmenter la pression à l'intérieur des ballonnets des valves 9 et 3, donc de fermer ces valves, et d'arrêter l'échappement du gaz sous pression distribué par la première source de débit 1;
- fournir une valeur de tension de démarrage à la première source de débit 1 au moins égale à valeur de tension de la turbine en fin de phase d'inspiration précédente, ce qui permet de minimiser le temps de montée en pression de la branche inspiratoire 2;
- et diminuer la valeur de la tension de la deuxième source de débit 4 soit jusqu'à une valeur nulle, soit jusqu'à une valeur préfixée, au choix.

Lorsque la tension de la deuxième source de débit 4 est maintenue à une valeur non nulle durant la phase inspiratoire, le gaz délivré par celle-ci est totalement échappé à l'atmosphère via l'échappement 11.

On comprend aisément que lors du passage de la phase expiratoire à la phase inspiratoire suivante, étant donné que la tension de commande de la première source de débit 1 n'est pas nulle, son inertie est réduite d'autant, ce qui permet une augmentation de pression rapide dans la branche inspiratoire 2.

Ainsi que schématisé sur la figure 3, lors de la détection de l'expiration (D. E.), on assiste, d'une part, au maintien de la tension de commande (Vt) de la première source de débit respiratoire à une valeur proportionnelle (coefficient de proportionnalité K') à la valeur moyenne de tension de commande (Vtm) de ladite première source de débit, mémorisée lors de la phase inspiratoire précédente, et, d'autre part, après basculement de l'électrovanne 8, au maintien de la tension de commande (Vp) de la deuxième source de débit respiratoire à une valeur proportionnelle non seulement à la différence entre la pression expiration positive (Ppep) et la pression mesurée (Pmes), mais aussi à l'intégrale de la dérivée de ladite différence entre Ppep et Pmes, pendant le temps d'expiration, (coefficients de proportionnalité respectifs: K" et I) . La valeur de tension de commande moyenne mémorisée (Vpm) de la deuxième source de débit est égale à Vp.

Sur les figures 2 et 3, les coefficients de proportionnalité K, K" et I sont aisément déterminables par l'homme du métier; ils seront choisis de manière à obtenir une régulation satisfaisante, c'est-à-dire notamment stable et rapide.

Ces différents coefficients dépendent de paramètres tels la longueur des conduits, les caractéristiques des matériels...

Par ailleurs, lorsqu'on souhaite obtenir une montée en pression rapide de la première source de débit, pendant les phases inspiratoires, on choisira de préférence un coefficient K' égal à 1.

A l'inverse, lorsqu'une montée plus lente en pression sera désirée, on fixera un coefficient K' inférieur à 1.

## Revendications

1. Dispositif d'assistance respiratoire comprenant :
- une branche inspiratoire (2) reliée en permanence, à son extrémité amont, à une première source de débit (1) de gaz sous pression et, à son extrémité aval, aux voies respiratoires d'un utilisateur,
- au moins une première (9) et une deuxième (3) valves expiratoires agencées sur ladite branche respiratoire (2) commandées pour être fermées pendant la phase inspiratoire,
- un moyen de détection (10) détectant, à proximité de l'extrémité aval de la branche inspiratoire, l'activité respiratoire de l'utilisateur et transmettant une donnée d'activité respiratoire à des moyens de pilotage (12), lesdits moyens de pilotage (12) commandant la première source de débit (1) de gaz de manière à délivrer, dans la branche inspiratoire (2), un flux de gaz à débit non nul et sensiblement constant pendant toute la phase expiratoire.

2. Dispositif selon la revendication 1, caractérisé en ce que les moyens de pilotage (12) commandent la première source de débit (1) de gaz de manière à délivrer, dans la branche inspiratoire (2), un flux de gaz dont le débit, pendant une phase expiratoire, est sensiblement égal au débit délivré en fin de phase inspiratoire précédente.

3. Dispositif selon l'une des revendications 1 ou 2, caractérisé en ce qu'au moins une première valve expiratoire (9) est agencée à proximité de l'extrémité amont de la branche inspiratoire (2).

4. Dispositif selon l'une des revendications 1 à 3, caractérisé en ce qu'au moins une deuxième valve expiratoire (3) est agencée à proximité de l'extrémité aval de la branche inspiratoire (2).

5. Dispositif selon l'une des revendications 1 à 4, caractérisé en ce qu'il comporte en outre une deuxième source de débit (4) de gaz sous pression et un moyen de distribution (8), ladite deuxième source de débit (4) et ledit moyen de distribution (8) étant commandés par les moyens de pilotage (12).

6. Dispositif selon la revendication 5, caractérisé en ce que le moyen de détection (10) est un capteur de pression.

7. Dispositif selon la revendication 5, caractérisé en ce que les valves expiratoires sont des valves pneumatiques à ballonnets.

8. Dispositif selon la revendication 5, caractérisé en ce que le moyen de distribution (8) est une électrovanne.

9. Dispositif selon l'une des revendication 1 à 8, caractérisé en ce que, durant la phase inspiratoire, l'électrovanne (8) établit la communication entre la première source de débit (1) de gaz sous pression et les ballonnets des valves pneumatiques, de manière à fermer lesdites valves pendant toute la phase inspiratoire.

10. Dispositif selon l'une des revendication 1 à 9, caractérisé en ce que, durant la phase expiratoire, l'électrovanne (8) établit la communication entre la deuxième source de débit (4) de gaz sous pression et les ballonnets des valves pneumatiques, de manière à contrôler l'ouverture desdites valves.

11. Dispositif selon l'une des revendications 9 ou 10, caractérisé en ce que le débit de la deuxième source (4) de gaz est inférieur au débit de la première source (1) de gaz sous pression.

12. Dispositif selon la revendication 11, caractérisé en ce qu'une partie du gaz délivré par la deuxième (4) source de gaz sous pression est échappé à l'atmosphère via un échappement (11).

13. Dispositif selon l'un des revendications précédentes, caractérisé en ce que le gaz sous pression est de l'air ou de l'air enrichi en oxygène.

14. Procédé de mise en oeuvre du dispositif selon l'une des revendications précédentes, caractérisé en ce que la tension de commande de la première source de débit (1) est maintenue à une valeur sensiblement constante et non nulle pendant toute la phase expiratoire.

15. Procédé selon la revendication 14, caractérisé en ce que la tension de commande de la première source de débit (1) est maintenue, pendant toute la phase expiratoire, à une valeur sensiblement égale à la valeur qu'elle avait en fin de phase inspiratoire précédente.

16. Procédé selon la revendication 15, caractérisé en ce que l'expiration est réalisée sous pression expiratoire positive.
